# EUROPÄISCHE PATENTSCHRIFT

(11) **EP 1 206 447 B1**
(45) Veröffentlichungstag und Bekanntmachung des Hinweises auf die Patenterteilung: **25.02.2004**
(21) Anmeldenummer: 00969245.0
(22) Anmeldetag: 23.08.2000
(51) Int. Cl.: C07C 279/18, A61K 31/155

(54) **SELEKTIVE INHIBITOREN DES UROKINASE-PLASMINOGEN AKTIVATORS**
SELECTIVE INHIBITORS OF THE UROKINASE PLASMINOGENE ACTIVATORS
INHIBITEURS SELECTIFS DE L'ACTIVATEUR DE L'UROKINASE PLASMINOGENE

(30) Priorität: 25.08.1999 DE 19940389
(43) Veröffentlichungstag der Anmeldung: 22.05.2002
(73) Patentinhaber: Wilex AG, 81675 München (DE)
(72) Erfinder: MAGDOLEN, Viktor, 85551 Kirchheim (DE); MORODER, Luis, 82152 Martinsried (DE); SPERL, Stefan, 82049 Pullach (DE); STÜRZEBECHER, Jörg, 99094 Erfurt-Rhoda (DE); WILHELM, Olaf, D-81545 München (DE)
(74) Vertreter: Weiss, Wolfgang, Dipl.-Chem. Dr.
(86) Internationale Anmeldenummer: PCT/EP2000/008234
(87) Internationale Veröffentlichungsnummer: WO 2001/014324

(56) Entgegenhaltungen:
- WO-A-99/20608
- DE-C- 947 552
- US-A- 3 257 411
- US-A- 5 914 319
- HEECHUNG YANG ET AL: "SELECTIVE INHIBITION OF UROKINASE BY SUBSTITUTED PHENYLGUANIDINES: QUANTITATIVE STRUCTURE-ACTIVITY RELATIONSHIP ANALYSES" JOURNAL OF MEDICINAL CHEMISTRY,US,AMERICAN CHEMICAL SOCIETY. WASHINGTON, Bd. 33, Nr. 11, 1990, Seiten 2956-2961, XP002059264 ISSN: 0022-2623
- R RAI, J A KATZENELLENBOGEN: "Guanidinophenyl-substituted enol lactones as selective, mechanism-based inhibitors of trypsin-like serine proteases" JOURNAL OF MEDICINAL CHEMISTRY., Bd. 35, Nr. 22, 1992, Seiten 4150--9, XP000978771 AMERICAN CHEMICAL SOCIETY. WASHINGTON., US ISSN: 0022-2623
- CHEMICAL ABSTRACTS, vol. 69, no. 9, 26. August 1968 (1968-08-26) Columbus, Ohio, US; abstract no. 35691c, BOLLAG, WERNER ET AL: "Substituted Benzylhydrazines" Seite 3321; Spalte 2; XP002157508 & CH 441 366 A (HOFFMANN LA ROCHE, F., AND CO., AG) 15. Januar 1968 (1968-01-15)

## Beschreibung

Die vorliegende Erfindung betrifft neue selektive Inhibitoren des Urokinase-Plasminogenaktivators (uPA, EC 3.4.21.31) vom Arylguanidintyp.

Der Plasminogenaktivator von Urokinase-Typ (uPA) spielt eine Schlüsselrolle bei der Tumorinvasion und Metastasenbildung (Schmitt et al., J. Obst. Gyn. 21 (1995), 151-165). uPA wird in verschiedenen Arten von Tumorzellen überexprimiert (Kwaan, Cancer Metastasis Rev. 11 (1992), 291-311) und bindet an den Tumor-assoziierten uPA-Rezeptor (uPA-R), wo die Aktivierung von Plasminogen zu Plasmin stattfindet. Plasmin ist in der Lage, verschiedene Komponenten der extrazellulären Matrix (ECM) wie Fibronectin, Laminin und Kollagen Typ IV abzubauen. Es aktiviert auch einige andere ECM-abbauende Enzyme, insbesondere Matrix-Metalloproteinasen. Hohe Mengen an Tumor-assoziiertem uPA korrelieren mit einem höheren Metastasierungsrisiko für Krebspatienten (Stephens et al., Breast Cancer Res. & Treat. 52 (1998), 99-111). Eine Hemmung der proteolytischen Aktivität von uPA ist daher ein guter Ansatzpunkt für eine anti-metastatische Therapie.

Ein gemeinsames Merkmal vieler bekannter synthetischer uPA-Inhibitoren ist ein basischer Rest, der Amidino- oder Guanidino-Gruppen enthält, und an Asp¹⁸⁹ in der S1-Spezifitätstasche von uPA binden kann und dort als Arginin-Mimetikum wirkt (Spraggon et al., Structure 3 (1995), 681-691). Die meisten der bekannten Inhibitoren sind jedoch nicht selektiv für uPA, sondern hemmen auch andere Serinproteasen wie Trypsin, Thrombin, Plasmin oder Gewebs-Plasminogenaktivator (tPA).

p-Aminobenzamidin ist ein moderat selektiver uPA-Inhibitor mit einer Hemmkonstante von 82 *µ*M. Billstroem et al. (Int. J. Cancer 61 (1995), 542-547) konnten eine deutliche Abnahme der Wachstumsrate von DU145 Tumoren (eine Prostata-Adenokarzinom-Zellinie) in SCID Mäusen bei oraler Verabreichung in einer Tagesdosis von 125 bis 250 mg p-Aminobenzamidin/kg/Tag zeigen. Die Nebenwirkungen waren vernachlässigbar gering.

Einige monosubstituierte Phenylguanidine haben sich als wirksame und selektive uPA Inhibitoren in vitro erwiesen. Diese kleinen Moleküle zeigen Inhibierungskonstanten im Mikromolarbereich, sie binden jedoch nur in der S1 Tasche von uPA (Yang et al., J. Med. Chem. 33 (1990), 2956-2961). Biologische Untersuchungen mit diesen Verbindungen wurden nicht durchgeführt.

Das Diuretikum Amilorid ist ein selektiver uPA-Inhibitor (Ki, uPA = 7 *µ*M), der die Bildung von Lungenmetastasen nach i.v. Inokulation von Ratten-Brustadenokarzinomzellen verhindert (Kellen et al., Anticancer Res. 8 (1988), 1373-1376). Einige Derivate von 3-Amidino-phenylalanin haben sich ebenfalls als wirksame Inhibitoren von Serinproteasen erwiesen, diese Verbindungen weisen jedoch im allgemeinen nur eine geringe Selektivität für uPA auf (Stürzebecher et al., J. Med. Chem. 40 (1997), 3091-3099; Stürzebecher et al., J. Enzyme Inhib. 9 (1995), 87-99).

WO 99/20608 offenbart tsochinolinguanidin-Derivate und deren Verwendung als uPA-Inhibitor. Im Gegensatz zu den erfindungsgemäßen Arylguanidin-Derivaten enthalten die Verbindungen gemäß WO 99/20608 einen bicyclischen aromatischen lsochinolinring als Grundgerüst.

In US-Patent Nr. 5,914,319 werden Thrombin-Inhibitoren offenbart, die Phenylguanidin-Gruppen enthalten können. Die Substitution am Phenylring der Verbindungen gemäß US-Patent Nr. 5,914,319 unterscheidet sich wesentlich von der der Verbindungen gemäß vorliegender Erfindung und eine Verwendung der in US-Patent Nr. 5,914,319 offenbarten Verbindungen als uPA-Inhibitoren wird nicht offenbart.

DE-C-947 552 offenbart Arylguanidin-Derivate, die durch die unterschiedliche Substitution an der Arylgruppe grundsätzlich von den erfindungsgemäßen Verbindungen verschieden sind. Die Verbindungen gemäß DE-C-947 552 werden als Wirkstoff gegen durch verschiedenartige Blutparasiten verursachte Infektionskrankheiten eingesetzt, eine Verwendung derselben als uPA-Inhibitoren wird nicht beschrieben.

US-Patent Nr. 3,257,411 offenbart die Verbindung 5-Methyl-3-(pguanidinophenyl)-4-isoxazolylcarbonsäure und deren Verwendung als Antibiotikum. Diese Verbindung unterscheidet sich grundsätzlich von der Struktur der erfindungsgemäßen Arylguanidin-Derivate.

In J. Med. Chem. **1992**, 32, 4150-4159 werden Guanidinophenylsubstituierte Enollactone als Inhibitoren von Serinproteasen, u.a. auch uPA, offenbart. Ein Hinweis auf die erfindungsgemäßen Verbindungen und die uPA-inhibierende Aktivität derselben findet sich in diesem Dokument nicht.

Chem. Abstr. 1968, 69, 35691 c offenbart substituierte Benzylhydrazine, die gegebenenfalls eine Guanidingruppe enthalten können. Einen Hinweis auf die erfindungsgemäß substituierten Arylguanidin-Derivate und auf die Verwendung von Arylguanidin-Derivaten als uPA-Inhibitoren findet sich in dieser Literaturstelle nicht.

Die derzeit wirksamsten und selektivsten uPA-Inhibitoren sind Derivate von Benzo[b]thiophen-2-carboxamidin (B428 und B623: Kᵢ, _{uPA} = 0,32 bzw. 0,07 *µ*M; US-Patent 5,340,833). Rabbani et al. (Int. J. Cancer 63 (1995), 840-845) sowie Xing et al. (Cancer Res. 57 (1997), 3585-3593) konnten nach Verabreichung von 4-lod-benzo[b]-thiophen-2-carboxamidin (B428) eine Abnahme des Tumorwachstums und der Metastasenbildung in einem syngenen Modell für Ratten-Prostatakarzinom bzw. Maus-Mammakarzinom zeigen. Letztere Untersuchungen zeigten eine weitere Abnahme des Primärtumorwachstums bei gemeinsamer Verabreichung von B428 mit dem Antiöstrogen Tamoxifen.

Die der vorliegenden Erfindung zugrundeliegende Aufgabe bestand darin, neue selektive uPA-Inhibitoren bereitzustellen. Diese Aufgabe wird durch neue Arylguanidin- und insbesondere Phenylguanidin-Derivate gelöst. Diese Verbindungen enthalten einen weiteren Substituenten am aromatischen Ringsystem, vorzugsweise in Para-Position zur Guanidingruppe, der eine gegebenenfalls substituierte Methylengruppe gefolgt von Wasserstoffdonor/Akzeptorfunktionalitäten enthält. Aufgrund dieses Substitutionsmusters weisen die Verbindungen eine besonders hohe Wirksamkeit und Selektivität für uPA auf. Diese Wirksamkeit könnte möglicherweise darauf zurückzuführen sein, daß sie
(1) als Arginin-Mimetikum mit dem Aminosäurerest Asp¹⁸⁹ in der S1-Tasche von uPA wechselwirken und
(2) eine Wechselwirkung mit der S2- und/oder S3-Tasche von uPA eingehen können.

N-substituierte p-Aminophenylguanidine (ohne Methylenspacer) sowie Derivate von p-Guanidino-phenylalanin (2 Methylengruppen als Spacer) waren als uPA-Inhibitoren unwirksam. Vorzugsweise enthalten die erfindungsgemäßen Verbindungen Urethan- oder Harnstoffgruppen für eine Wechselwirkung mit S2 und/oder große hydrophobe Reste wie Arylgruppen oder Cycloalkylgruppen (z.B. Adamantan) für eine Wechselwirkung mit S3.

Ein Gegenstand der vorliegenden Erfindung ist somit die Verwendung von Verbindungen der allgemeinen Formel I 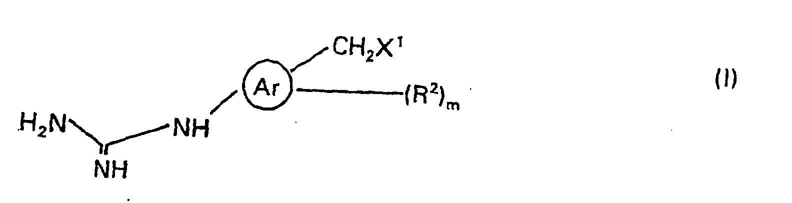 worin
- Ar: ein aromatisches oder heteroaromatisches Ringsystem mit einem einzigen Ring bedeutet;
- X¹: NR³R ⁴, OR³, SR³, COOR³, CONR³R⁴ oder COR⁵ bedeutet,
wobei
R³ H oder eine Gruppe der allgemeinen Formel II, IIIa, IIIb oder IIIc bedeutet: 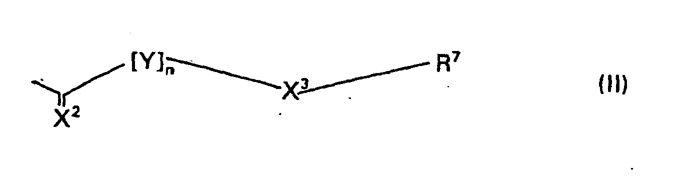 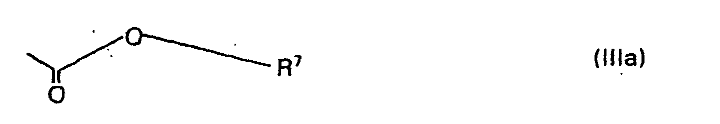 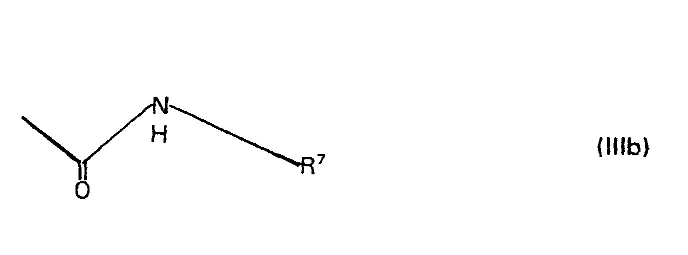 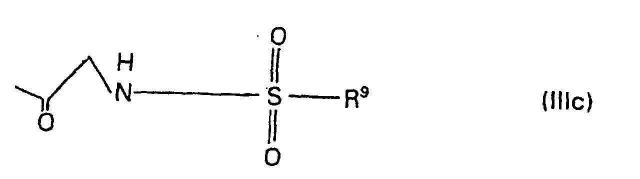 wobei
X² NH, NR⁴, O oder S bedeutet,
X³ NH, NR⁴, O, S, CO, COO, CONH oder CONR⁴ bedeutet,
Y C(R⁸)₂ bedeutet,
R⁴ H oder einen Alkyl-, Alkenyl- oder Alkinyl-Rest bedeutet,
R⁷ H oder einen Alkyl-, Alkenyl-, Alkinyl-, Aryl-, Heteroaryl- oder/und Cycloalkylrest oder -SO₂-R⁹ bedeutet,
R⁸ jeweils unabhängig H, Halogen oder einen Alkyl-, Alkenyl-, Alkinyl-, Aryl- oder/und Heteroarylrest bedeutet,
R⁹ H oder einen Alkyl-, Alkenyl-, Alkinyl-, Aryl-, Heteroaryl- oder/und Cycloalkylrest bedeutet und
n eine ganze Zahl von 0 bis 2 ist,
R⁴ wie oben definiert ist,
R⁵ H, einen Alkyl-, Alkenyl-, Alkinyl-, Carboxy-alkyl-, Carboxy-alkenyl-, Carboxy-alkinyl-, Carboxy-aryl- oder Carboxy-heteroarylrest bedeutet;
R² Halogen, C(R⁶)₃, C₂(R ⁶)₅, OC(R⁶)₃ oder OC₂(R⁶)₅ bedeutet,
wobei
R⁶ jeweils unabhängig H oder Halogen, insbesondere F, ist; und
- m: eine ganze Zahl von 0 bis 4 ist;
oder Salzen dieser Verbindungen zur Herstellung eines Mittels zur Hemmung des Urokinase-Plasminogenaktivators.

Die Verbindungen können als Salze, vorzugsweise als physiologisch verträgliche Säuresalze, z.B. als Salze von Mineralsäuren, besonders bevorzugt als Hydrochloride oder als Salze von geeigneten organischen Säuren vorliegen. Die Guanidiniumgruppe kann gegebenenfalls Schutzfunktionen tragen, die vorzugsweise unter physiologischen Bedingungen abspaltbar sind. Die Verbindungen können als optisch reine Verbindungen oder als Gemische von Enantiomeren oder/und Diastereoisomeren vorliegen.

In den Verbindungen der allgemeinen Formel (I) ist Ar ein aromatisches oder heteroaromatisches Ringsystem mit einem einzigen Ring, insbesondere ein Benzolring. In diesem Ringsystem sind die Substituenten·CH₂X¹ und NHC(NH)NH₂ vorzugsweise in Meta- oder Para-Position und besonders bevorzugt in Para-Position zueinander angeordnet. Darüber hinaus kann Ar noch weitere von Wasserstoff verschiedene Substituenten R² enthalten. Vorzugsweise ist die Anzahl der Substituenten R² 0, 1, 2 oder 3, besonders bevorzugt 0 oder 1 und am meisten bevorzugt 0. Bevorzugte Beispiele für R² sind Halogenatome (F, Cl, Br oder 1), CH₃, CF₃, OH, OCH₃ oder OCF₃.

Für die Inhibitoraktivität kritisch ist der Substituent CH₂X¹.

Die Gruppe X¹ ist ein Rest mit Elektronendonor- oder/und Elektronenakzeptor-Eigenschaften, wie NR³R⁴, OR³, SR³, COOR³, CONR³R⁴ oder COR⁵. Besonders bevorzugt ist X¹ NR³R⁴. R³ kann Wasserstoff oder eine Gruppe der allgemeinen Formel II, IIIa, IIIb oder IIIc bedeuten, 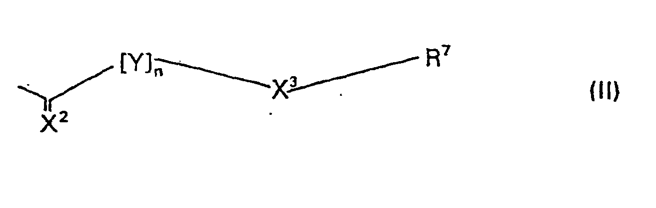 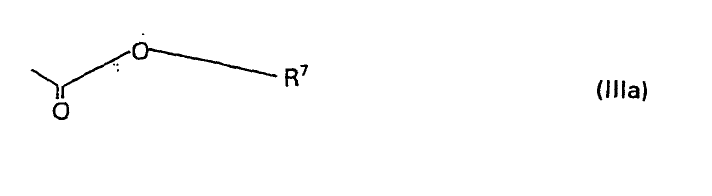 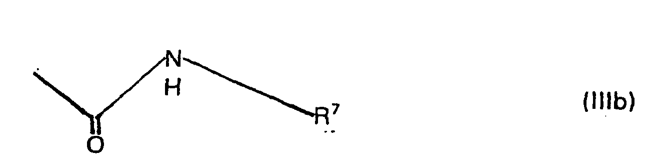 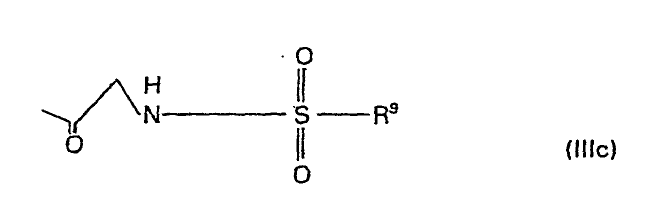 wobei
- X²: NH, NR⁴, O oder S bedeutet,
- X³: NH, NR⁴, O, S, CO, COO, CONH oder CONR⁴ bedeutet,
- Y: C(R⁸)₂ bedeutet,
- R⁴: H oder einen Alkyl-, Alkenyl- oder Alkinyl-Rest bedeutet,
- R⁷: H oder einen Alkyl-, Alkenyl-, Alkinyl-, Aryl-, Heteroaryloder/und Cycloalkylrest oder -SO₂-R⁹ bedeutet,
- R⁸: jeweils unabhängig H, Halogen oder einen Alkyl-, Alkenyl-, Alkinyl-, Aryl- oder/und Heteroarylrest bedeutet,
- R⁹: H oder einen Alkyl-, Alkenyl-, Alkinyl-, Aryl-, Heteroaryloder/und Cycloalkylrest bedeutet und
- n: eine ganze Zahl von 0 bis 2 ist.

R⁵ kann Wasserstoff, ein Alkyl-, Alkenyl-, Alkinyl-, Carboxy-alkyl-, Carboxyalkenyl-, Carboxy-alkinyl-, Carboxy-aryl-oderCarboxy-heteroaryl-Restsein. Vorzugsweise ist R⁵ ein raumfüllender Rest und enthält mindestens eine Aryl-, Heteroaryl-, Cycloalkyl- oder/und tert.-Alkylgruppe. Besonders bevorzugt sind Phenylreste, substituierte Phenylreste, tert. Alkylreste und Cycloalkylreste, die gegebenenfalls Substituenten wie vorstehend definiert enthalten können.

Besonders bevorzugt ist R³ eine Gruppe der allgemeinen Formel (II): 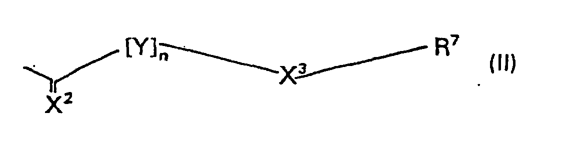 worin
- X²: NH, NR⁴, O oder S bedeutet,
- X³: NH, NR⁴, O, S, CO, COO, CONH oder CONR⁴ bedeutet,
- Y: C(R⁸)₂ bedeutet,
- R⁴: wie in Formel (I) definiert ist,
- R⁷: H oder einen gegebenenfalls substituierten Alkyl-, Alkenyl-, Alkinyl-, Aryl- oder/und Heteroarylrest oder -SO₂-R⁹ bedeutet,
- R⁸: jeweils unabhängig H, Halogen oder einen gegebenenfalls substituierten Alkyl-, Alkenyl-, Alkinyl- oder Aryl- oder/und Heteroarylrest bedeutet,
- R⁹: H oder einen gegebenenfalls substituierten Alkyl-, Alkenyl-, Alkinyl-, Aryl- oder/und Heteroarylrest bedeutet und
- n: eine ganze Zahl von 0 bis 2 ist.

X² ist vorzugsweise NH oder 0, besonders bevorzugt O. X³ ist vorzugsweise NH oder -O-. Y ist vorzugsweise CH₂ oder CHR⁸, wobei R⁸ vorzugsweise wie R⁴ in Formel (I) definiert ist.

R⁷ und R⁹ sind vorzugsweise wie R⁵ in Formel (I) definiert.

Am meisten bevorzugt ist R³ eine Gruppe der allgemeinen Formeln IIIa, IIIb oder IIIc: 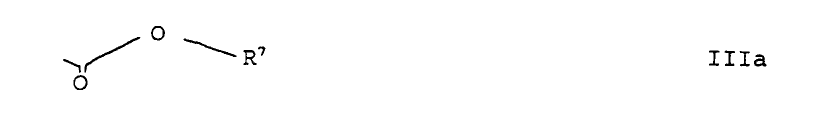 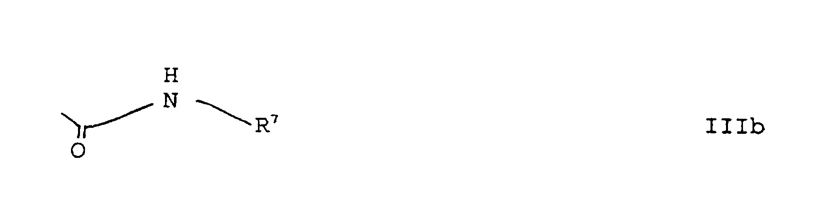 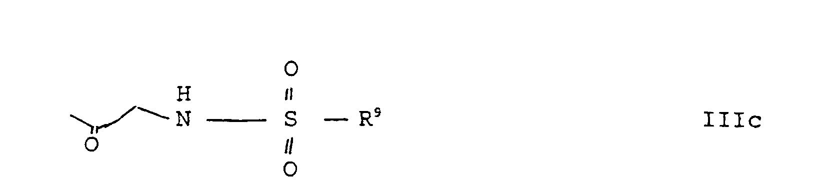 worin R⁷ und R⁹ wie in Formel (II) definiert sind.

Die Substituenten R⁷ und R⁹ enthalten - ebenso wie R⁵ - vorzugsweise raumfüllende Gruppen, die ausgewählt sein können aus gegebenenfalls substituierten Arylresten, insbesondere Phenyl- und substituierten Phenylresten und gegebenenfalls substituierten verzweigte Alkyl-, Alkenyloder Alkinylresten, insbesondere mit tertiären C-Atomen wie tert.-Butyl oder Neopentyl oder gegebenenfalls substituierten Cycloalkylresten, insbesondere Bi- oder Tricycloalkylresten wie Adamantyl.

Eine besonders hohe Affinität und Selektivität für uPA haben auch Verbindungen der allgemeinen Formel (IV): 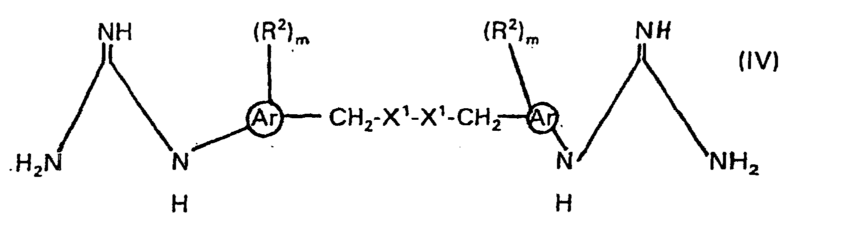 worin
- X¹: jeweils unabhängig NR³R⁴, OR³, SR³, COOR³, CONR³R⁴ oder COR⁵ bedeutet, mit der Maßgabe, dass die zwei Arylguanidingruppen über die Substituenten CH₂X¹ miteinander verknüpft sind,
wobei
R³ jeweils unabhängig H oder einen beliebigen organischen Rest bedeutet,
R⁴ jeweils unabhängig H oder einen Alkyl-, Alkenyl- oder Alkinyl-Rest bedeutet,
Ar jeweils unabhängig ein aromatisches oder heteroaromatisches Ringsystem bedeutet,
R² jeweils unabhängig Halogen, C(R⁶)₃, C₂(R ⁶)₅, OC(R ⁶)₃ oder OC₂(R⁶)₅ bedeutet, wobei
- R⁶: jeweils unabhängig H oder Halogen, insbesondere F, ist, und
- m: eine ganze Zahl von 0 bis 4 ist, oder Salze dieser Verbindungen.

Die Verbindungen der Formel (IV) enthalten zwei Arylguanidinogruppen und sind über ihre Substituenten CH₂X¹-, die jeweils gleich oder verschieden sein können, miteinander verknüpft.

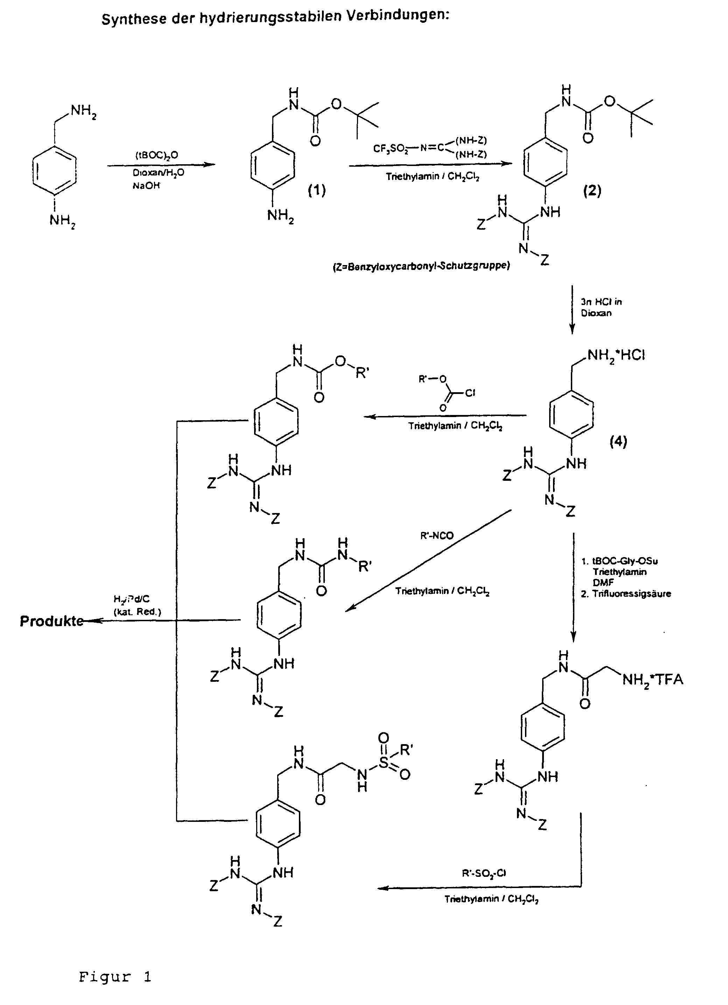

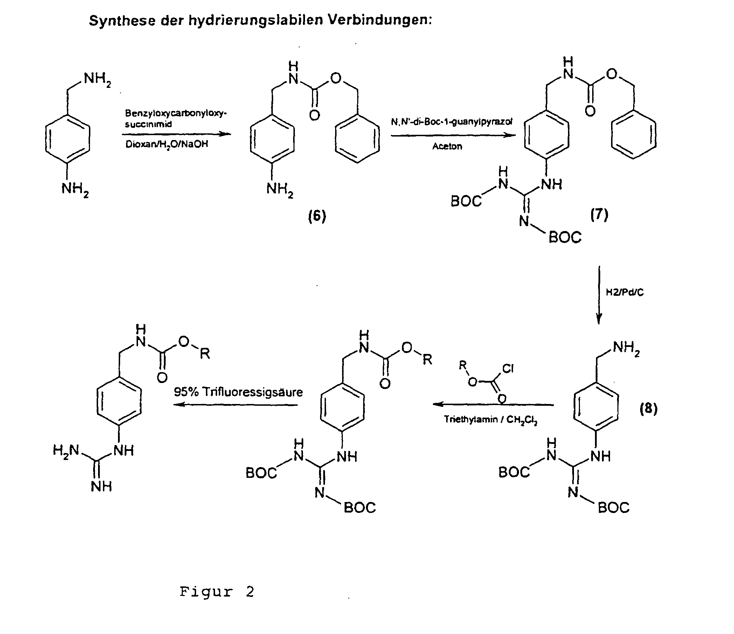

Die Verbindungen der allgemeinen Formel (I) können beispielsweise ausgehend von ρ-Amino-benzylamin gemäß den in den Figuren 1 und 2 gezeigten Reaktionsschematen hergestellt werden. 4-Amino-benzylamin kann beispielsweise mit einem Schutzreagenz für Aminogruppen, z.B. Di-tert-butyl-pyrocarbonat zu einem geschützten Zwischenprodukt 4-(N-Boc-Aminomethyl)-anilin (1) umgesetzt werden, wobei Boc tert-Butyloxycarbonyl bedeutet. Die aromatische Aminofunktion dieser Verbindung kann mit einem Guanidinylierungsreagenz, z.B. N,N'-di-Z-N"triflylguanidin umgesetzt werden, wobei 1-[4-(N-Boc-aminomethyl)-phenyl]-2,3-di-Z-guanidin (2) entsteht, wobei Z Benzyloxycarbonyl bedeutet. Diese Verbindung kann durch Abspaltung der Boc-Schutzgruppe zu 1-[4-(Aminomethyl)-pheny)]-2,3-di-Z-guanidinium-hydrochlorid (4) umgesetzt werden. Die Verbindung (4) kann wiederum mit reaktiven Verbindungen wie etwa Chlorameisensäureestern, Isocyanaten oder N-Hydroxysuccinimidestern zu den gewünschten Endprodukten umgesetzt werden.

Die Darstellung hydrierungslabiler Verbindungen ist in Figur 2 beschrieben. 4-Amino-benzylamin kann mit einem Schutzreagenz für Aminogruppen, z.B. Benzyloxycarbonyloxy-succinimid zu einem geschützten Zwischenprodukt (6) und dann mit einem weiteren Guanidinylierungsreagenz, z.B. N,N'-di-Boc-1-guanylpyrazol zu (7) umgesetzt werden. Diese Verbindung kann zu (8) hydriert und anschließend mit reaktiven Verbindungen zu den gewünschten Endprodukten umgesetzt werden.

Auf entsprechende Weise können auch Verbindungen synthetisiert werden, bei denen X¹ die Bedeutung OR³, SR³, COOR³, CONR³R⁴ oder COR⁵ hat.

Die erfindungsgemäßen Urokinaseinhibitoren können gegebenenfalls zusammen mit geeigneten pharmazeutischen Hilfs- oder Trägerstoffen zur Herstellung von Arzneimitteln oder in der Diagnostik verwendet werden. Dabei ist eine Verabreichung in Kombination mit anderen Wirkstoffen, z.B. anderen Urokinaseinhibitoren wie etwa Antikörpern oder/und Peptiden möglich.

Die Arzneimittel können bei Menschen und Tieren topisch, oral, rektal oder parenteral, z.B. subkutan oder intravenös, z.B. in Form von Tabletten, Dragees, Kapseln, Pellets, Suppositorien, Lösungen oder transdermalen Systemen, wie Pflastern, verabreicht werden.

Die erfindungsgemäßen Verbindungen sind zur Bekämpfung von Krankheiten geeignet, die mit einer pathologischen Überexpression von uPA oder/und uPAR assoziiert sind. Sie sind beispielsweise in der Lage, hocheffizient das Wachstum oder/und die Ausbreitung der malignen Tumoren sowie die Metastasierung von Tumoren zu hemmen. Dabei können die uPA-Inhibitoren gegebenenfalls zusammen mit anderen Tumormitteln oder mit anderen Behandlungsarten, z.B. Bestrahlung oder chirurgischen Eingriffen, eingesetzt werden. Weiterhin sind die erfindungsgemäßen Inhibitoren auch für andere uPA-assoziierte Erkrankungen wirksam.

Erfindungsgemäße uPA-Inhibitoren sind vorzugsweise dadurch gekennzeichnet, daß sie mindestens einen zweifach, vorzugsweise mindestens einen fünffach und besonders bevorzugt einen mindestens zehn- und bis zu 1000-fach geringeren Kᵢ-Wert für uPA gegenüber tPA aufweisen. Weiterhin ist bemerkenswert, daß die erfindungsgemäßen Verbindungen die Blutgerinnung nur geringfügig beeinflussen, da sie für eine effektive Hemmung von Thrombin, Plasmin und Faktor Xa zu hohe Kᵢ-Werte haben.

Die erfindungsgemäßen Substanzen der Formel (I) können in Form von Konjugaten mit physiologisch wirksamen Substanzen eingesetzt werden, z.B. mit Radiomarkierungen oder mit zytotoxischen Mitteln, z.B. Chemotherapeutika wie cis-Platin oder 5-Fluor-uracil, oder Peptiden. Weiterhin können die Substanzen auch in die Membran von Trägervesikeln, z.B. Liposomen, eingebaut werden und somit ein Targeting von in den Trägervesikeln eingeschlossenen Wirksubstanzen, z.B. zytotoxischen Mitteln, wie etwa Doxorubicin, ermöglichen.

Die Dosierung der Verbindung liegt üblicherweise im Bereich von 0,01 bis 100 mg/kg Körpergewicht pro Tag. Die Dauer der Behandlung hängt von der Schwere der Erkrankung ab und kann von einer einmaligen Gabe bis zu einer mehrwöchigen oder sogar mehrmonatigen Behandlung, die gegebenenfalls in Intervallen wiederholt werden kann, reichen.

Schließlich betrifft die Erfindung neue Aryl-Guanidinderivate der allgemeinen Formel (I).

Die Erfindung soll an den folgenden Beispielen und Abbildungen näher erläutert werden. Es zeigt:
- Figur 1: ein allgemeines Reaktionsschema zur Herstellung erfindungsgemäßer hydrierungsstabiler Substanzen.
- Figur 2: ein allgemeines Reaktionsschema zur Herstellung erfindungsgemäßer hydrierungslabiler Substanzen.

### Beispiele

### Material und Methoden

Alle für die Synthese von uPA-Inhibitoren verwendeten Lösungsmittel und Reagenzien waren von der höchsten kommerziell verfügbaren Qualität und wurden - sofern erforderlich - durch Standardmethoden weiter aufgereinigt und getrocknet. Die analytische HPLC erfolgte auf Nucleosil 100/C18 Säulen (Macherey-Nagel, Düren, Deutschland) unter Verwendung eines linearen Acetonitril/2% H₃PO₄ Gradienten (von 5:95 bis 90:10 in 13 min) ESI-MS-Spektren wurden auf einem Perkin Elmer API 165 Massenspektrometer gemessen.

### Beispiel 1 Synthese von säurelabilen Urethanen, z.B. 4-(N-Bocaminomethyl)-phenylguanidin (3)

### 4-(N-Boc-aminomethyl)-anilin (1)

4-Amino-benzylamin (2 ml; 17,6 mmol) wurde in 1,4-Dioxan (10 ml) gelöst. Unter Rühren wurde eine wässrige 2 N NaOH-Lösung (17,6 ml; 35,2 mmol) zugegeben. Eine Lösung von Di-tert-butyl-pyrocarbonat (3,08 g; 14,1 mmol) in 1,4-Dioxan (30 ml) wurde tropfenweise über 30 min zugegeben und das Reaktionsgemisch über Nacht bei Raumtemperatur gerührt. Die Lösung wurde im Vakuum auf etwa 10 ml konzentriert und zweimal mit Ethylacetat (30 ml) extrahiert. Die vereinigten organischen Phasen wurden mit wässrigem 5% KHSO₄ (10 ml), wässrigem 5% NaHCO₃, Wasser und Salzlösung gewaschen, über wasserfreiem Na₂SO₄ getrocknet und im Vakuum eingedampft, wobei das Produkt als hellgelbe Festsubstanz erhalten wurde.
- Ausbeute:: 2,38 g (76 %); HPLC: t_{R} 5,6 min; MS 223 (M + H) ⁺, berechnet 222 (M).

### 1-[4-(N-Boc-aminomethyl)-phenyl]-2,3-di-Z-guanidin (2)

Eine Lösung der Verbindung (1) (500 mg; 2,24 mmol) und N,N'-di-Z-N"triflylguanidin (1,04 g; 2,24 mmol) (Feichtinger et al., J. Org. Chem. 63 (1998), 3804-3805) in 5 ml Aceton wurde bei Raumtemperatur heftig gerührt. Nach 10 min begann das Produkt in Form eines Präzipitats auszufallen. Nach 2 h wurde das Produkt abfiltriert, im Vakuum getrocknet und aus Methanol umkristallisiert, wobei weiße Kristalle erhalten wurden.
- Ausbeute:: 1,065 g (89%); HPLC: t_{R} 13,4 min; MS 533 (M+H)⁺, berechnet 532 (M).

### 4-(Boc-aminomethyl)-phenylguanidinium-hydrochlorid (3)

50 mg (0,107 mmol) der Verbindung (2) wurden in 5 ml Methanol gelöst, gerührt und für 3 h über einem 10% Palladium-Aktivkohle-Katalysator hydriert. Nach Entfernung des Katalysators durch Filtration wurde das Lösungsmittel im Vakuum abgedampft. Der Rückstand wurde aus Methanol/Diisopropylether nach Zugabe von einem Äquivalent HCl in 1,4-Dioxan umkristallisiert.
- Ausbeute:: 28 mg (87%); HPLC, t_{R} 7,1 min; MS 265 (M + H)⁺, berechnet 264 (M).

### Beispiel 2 Synthese von disubstituierten Harnstoffen unter Verwendung von 1-[4-(Aminomethyl)-phenyl]-2,3-di-Z-guanidiniumhydrochlorid (4) als Baustein, z.B. 4-[3-(-1-Adamantyl)-ureido]-phenyl-guanidinium-hydrochlorid (5)

### 1-[4-(Aminomethyl)-phenyl]-2,3-di-Z-guanidinium-hydrochlorid (4)

1 g (1,878 mmol) der Verbindung (2) wurde in 20 ml 3 N HCl (Gas) in 1,4-Dioxan bei 0°C gelöst und für 2 h bei Raumtemperatur gerührt. Nach Abdampfen des Lösungsmittels wurde das kristalline Produkt in nahezu quantitativer Ausbeute erhalten.
- Ausbeute:: 872 mg (99%); HPLC: t_{R} 10,2 min; MS 433 (M+H)⁺, berechnet 432 (M).

### 4-[3-(-1-Adamantyl)-ureido]-phenyl-guanidinium-hydrochlorid (5)

50 mg (0,107 mmol) der Verbindung (4), 17 mg (0,107 mmol) Adamantylisocyanat und 45 *µ*l (0,32 mmol) Triethylamin wurden in 1 ml Ethylenchlorid gelöst. Das Reaktionsgemisch wurde für 3 h bei Raumtemperatur gerührt.

Nach Abdampfen des Lösungsmittels im Vakuum wurde der Rückstand in Ethylacetat (10 ml) gelöst und dreimal mit 0,1 N wässriger HCl extrahiert. Die organische Phase wurde bis zu Trockene eingedampft. Die Entfernung der Z-Schutzgruppen erfolgte wie für Verbindung (3) beschrieben.
- Ausbeute:: 15 mg (37%); HPLC: t_{R} 8,6 min; MS 342 (M + H) ⁺, berechnet 341 (M)

### Beispiel 3 Synthese von hydrierungslabilen Verbindungen, z.B. 4-[N-(4-Nitrobenzyloxycarbonyl)-aminomethyl]-phenylguanidin (9)

### 4-(N-Z-Aminomethyl)-anilin (6)

4-Amino-benzylamin (1 ml; 8,82 mmol) wurde in 10 ml 1,4-Dioxan gelöst. Eine wässrige 2 NaOH Lösung von NaOH (8,8 ml; 17,64 mol) wurde unter Rühren zugegeben. Dann wurde eine Lösung von Benzyloxycarbonyloxy-succinimid (1,978 g; 7,938 mmol) in 10 ml 1,4-Dioxan tropfenweise über 15 min zugegeben, und das Reaktionsgemisch für 5 h bei Raumtemperatur gerührt. Die Lösung wurde im Vakuum auf etwa 10 ml konzentriert und zweimal mit 30 ml Ethylacetat extrahiert. Die vereinigten organischen Phasen wurden mit wässriger 5%iger NaHCO₃ Lösung, Wasser und Salzlösung gewaschen, über wasserfreiem Na₂SO₄ getrocknet, eingedampft und im Vakuum getrocknet, wobei das Produkt als hellgelbe Festsubstanz erhalten wurde.
- Ausbeute:: 1,8 g (88%); HPLC: t_{R} 6,8 min; MS 257 (M + H)⁺, berechnet 256 (M).

### 1-[4-[N-Z-Aminomethyl)-phenyl)-2,3-di-Boc-guanidin (7)

Eine Lösung von 495 mg (1,93 mmol) der Verbindung (6) und 599 mg (1,93 mmol) N,N'-di-Boc-1-guanylpyrazol (Bernatowicz et al., Tetrahedron Lett. 34 (1993), 3389-3392) in 5 ml Aceton wurde für 3 Tage bei Raumtemperatur gerührt. Nach Abdampfen des Lösungsmittels wurde der Rückstand in 50 ml Diethylether gelöst, mit wässriger 5% KHSO₄ Lösung, Wasser und Salzlösung gewaschen und über wasserfreiem Na₂SO₄ getrocknet. Nach Abdampfen des Diethylethers im Vakuum wurde ein hellgelber Schaum erhalten.
- Ausbeute:: 670 mg (70%); HPLC: t_{R} 12,1 min; MS 499 (M+H)⁺, berechnet 498 (M)

### 1-(4-Aminomethyl)-phenyl-2,3-di-Boc-guanidin-hydrochlorid (8)

Die Verbindung (8) wurde durch katalytische Hydrierung von 600 mg (1,2 mmol) der Verbindung (7) in Ethanol über einem 10% Palladium-Aktivkohle-Katalysator für 1 h erhalten. Nach Filtration des Katalysators wurde das Lösungsmittel im Vakuum eingedampft, wobei ein Öl erhalten wurde, das aus Isopropanol/Diisopropylether nach Zugabe von 1 Äquivalent HCl in 1,4-Dioxan umkristallisiert wurde.
- Ausbeute:: 450 mg (91 %); HPLC: t_{R} 8,1 min; MS 365 (M + H) ⁺, berechnet 364 (M)

### 4-(N-(4-Nitrobenzyloxycarbonyt)-aminomethyl]-phenylguanidin-hydrochlorid (9)

Eine Lösung von 50 mg (0,125 mmol) der Verbindung (8), 27 mg (0,125 mmol) 4-Nitrobenzylchlorformiat und 52 *µ*l (0,375 mmol) Triethylamin in 1 ml Methylenchlorid wurde für 3 h bei Raumtemperatur gerührt. Nach Abdampfen des Lösungsmittels wurde der Rückstand in 30 ml Ethylacetat aufgelöst und dreimal mit 0,5 N wässriger HCl gewaschen. Nach Abdampfen des Ethylacetats wurde der Rückstand in 95% Trifluoressigsäure gelöst und für 1 h gerührt. Nach Abdampfen des Lösungsmittel wurde das Produkt aus Ethanol/Diisopropylether umkristallisiert.
- Ausbeute:: 35 mg (60%); HPLC: t_{R} 8,1 min; MS 344 (M+H)⁺, berechnet 343 (M).

### Beispiel 4 In vitro Hemmung von Urokinase durch ausgewählte Verbindungen der Formel I

Zur Bestimmung der uPA Inhibitoraktivität wurden 200 *µ*l Tris-Puffer (0,05 mol/l, den Inhibitor enthaltend, 0,154 mol/l NaCl, 5% Ethanol, pH 8,0), 25 µl Substrat (Pefachrome UK oder BZ-β-Ala-Gly-Arg-pNA in H₂O; Pentapharm LTD, Basel, Schweiz) und 50 µl sc-Urokinase (Ribosepharm GmbH, Haan, Deutschland), bzw. eine entsprechende andere Protease bei 25 °C inkubiert. Nach 3 min wurde die Reaktion durch Zugabe von 25 *µ*l Essigsäure (50%) unterbrochen und die Absorption bei 405 nm mittels eines Mikroplate Reader (MR 5000, Dynatech, Denkendorf, Deutschland) bestimmt. Die Kᵢ-Werte wurden nach Dixon durch lineare Regression mittels eines Computerprogramms ermittelt. Die Kᵢ-Werte sind das Mittel aus mindestens drei Bestimmungen, die Standardabweichung lag unter 25%. Die getesteten Inhibitoren und ihre Inhibitionskonstanten für verschiedene Proteasen sind in der folgenden Tabelle 1 dargestellt:

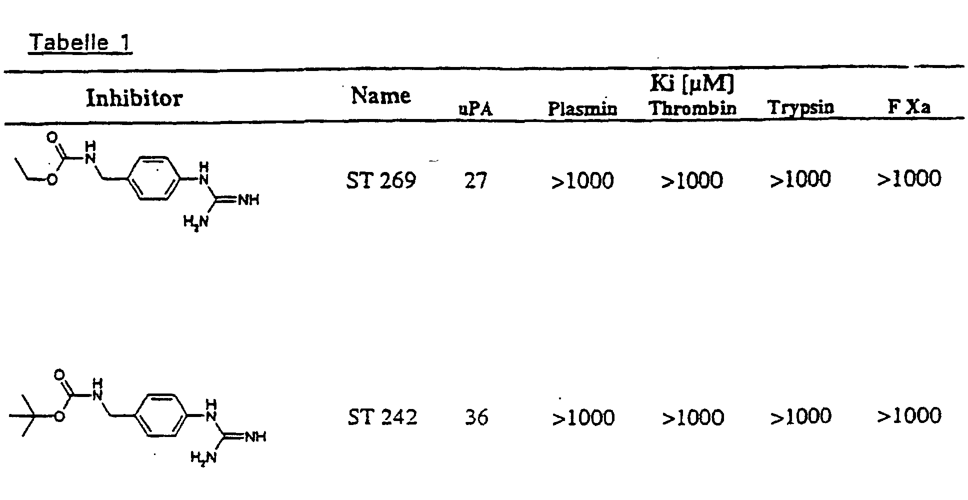

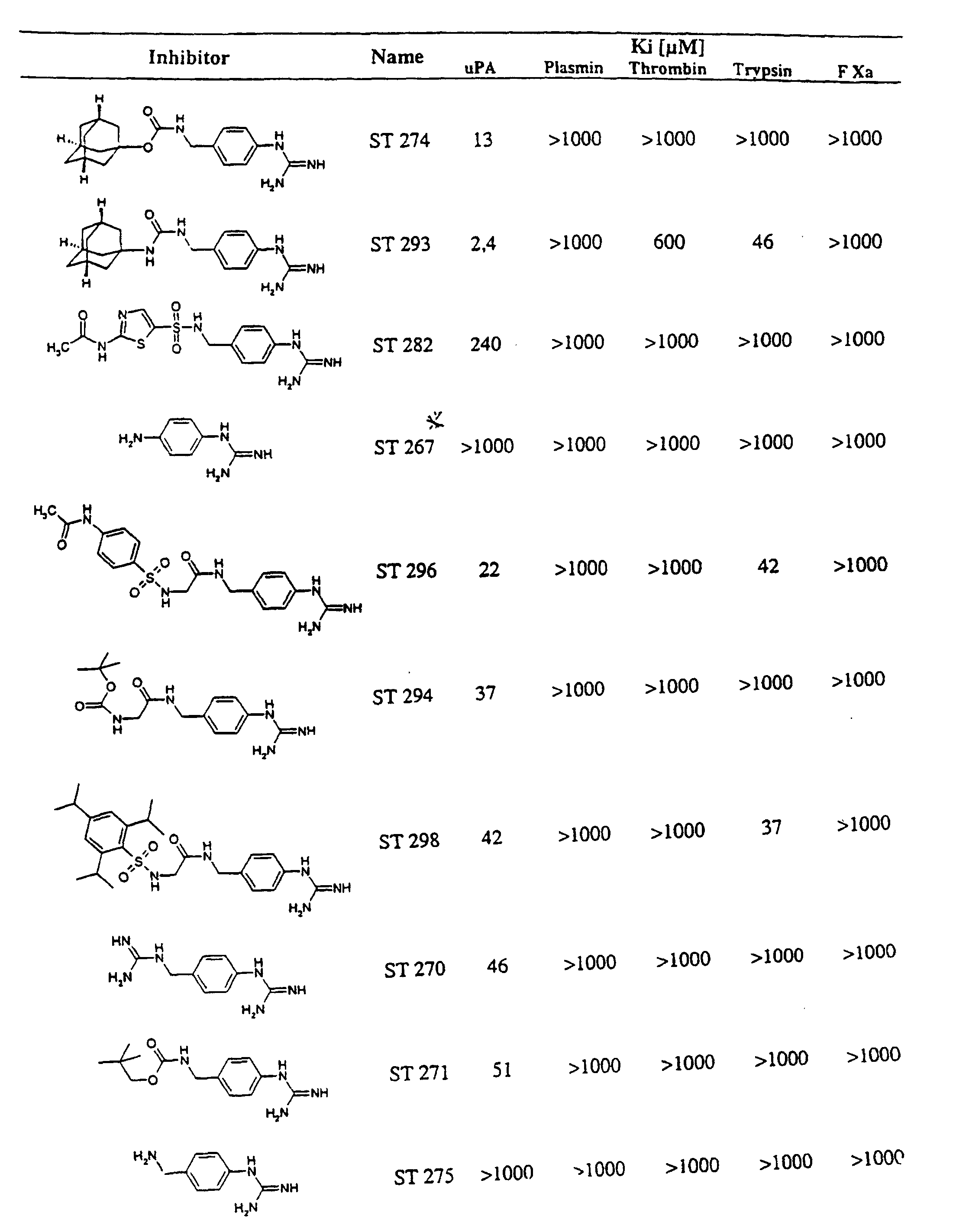

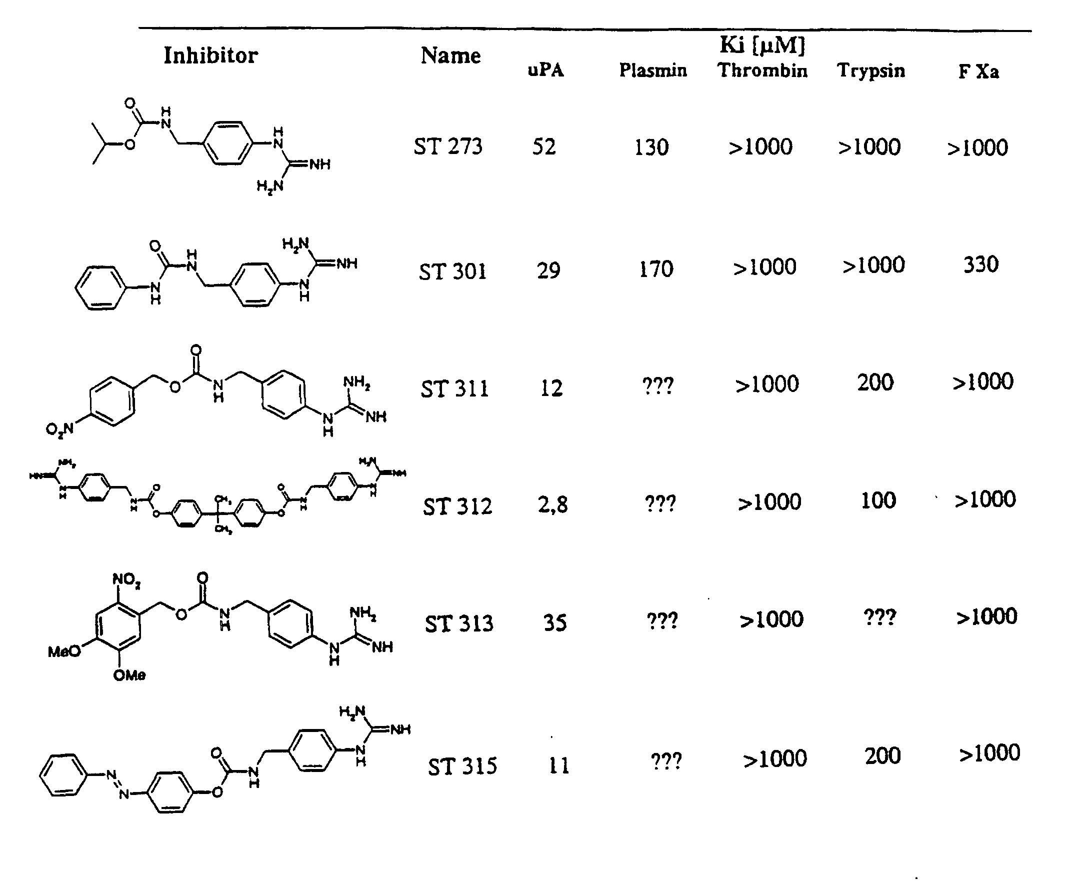

Die Verbindungen ST293, 312 und 315 weisen einen Ki-Wert für uPA von <11µM auf.

Die als ST293 und ST312 bezeichneten Verbindungen erwiesen sich als besonders wirksame und selektive Inhibitoren.

## Patentansprüche

1. Verwendung von Verbindungen der allgemeinen Formel (I) 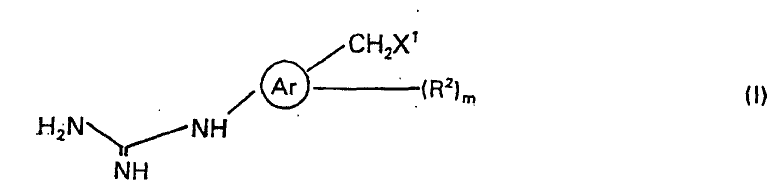 worin
Ar ein aromatisches oder heteroaromatisches Ringsystem mit einem einzigen Ring bedeutet;
X¹ NR³R⁴, OR³, SR³, COOR³, CONR³R⁴ oder COR⁵ bedeutet,
wobei
R³ H oder eine Gruppe der allgemeinen Formel II, IIIa, IIIb oder IIIc bedeutet: 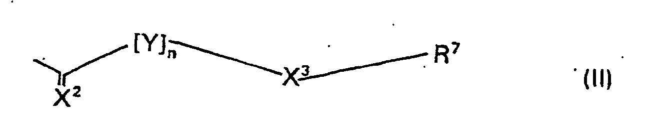 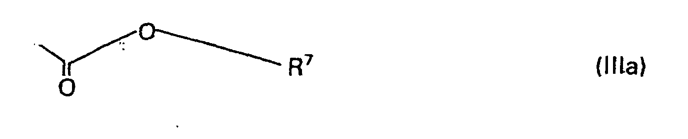 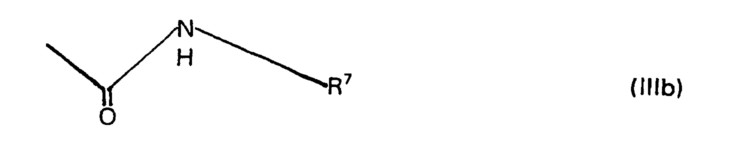 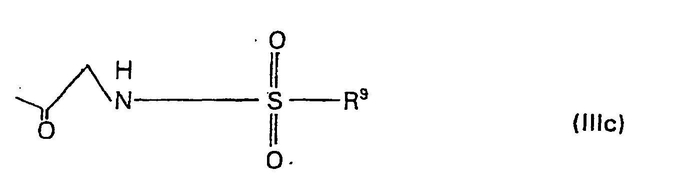 wobei
X² NH, NR⁴, O oder S bedeutet,
X³ NH, NR⁴, O, S, CO, COO, CONH oder CONR⁴ bedeutet,
Y C(R⁸)₂ bedeutet,
R⁴ H oder einen Alkyl-, Alkenyl- oder Alkinyl-Rest bedeutet,
R⁷ H oder einen Alkyl-, Alkenyl-, Alkinyl-, Aryl-, Heteroaryl- oder/und Cycloalkylrest oder -SO₂-R⁹ bedeutet,
R⁸ jeweils unabhängig H, Halogen oder einen Alkyl-, Alkenyl-, Alkinyl-, Aryl- oder/und Heteroarylrest bedeutet,
R⁹ H oder einen Alkyl-, Alkenyl-, Alkinyl-, Aryl-, Heteroaryl- öder/und Cycloalkylrest bedeutet und
n eine ganze Zahl von 0 bis 2 ist,
R⁴ wie oben definiert ist,
R⁵ H, einen Alkyl-, Alkenyl-, Alkinyl-, Carboxy-alkyl-, Carboxy-alkenyl-, Carboxy-alkinyl-, Carboxy-aryl- oder Carboxy-heteroarylrest bedeutet;
R² Halogen, C(R⁶)₃, C₂ (R⁶)₅, OC(R⁶)₃ oder OC₂(R⁶)₅ bedeutet,
wobei
R⁶ jeweils unabhängig H oder Halogen, insbesondere F, ist; und
m eine ganze Zahl von 0 bis 4 ist;
oder Salzen dieser Verbindungen zur Herstellung eines Mittels zur Hemmung des Urokinase-Plasminogenaktivators.

2. Verwendung nach Anspruch 1, worin Ar einen Benzolring bedeutet.

3. Verwendung nach Anspruch 2, worin die Substituenten -CH₂X¹ und - NHC(NH)NH₂ in para-Position zueinander angeordnet sind.

4. Verwendung nach einem der Ansprüche 1 bis 3, worin R⁷ und R⁹ ausgewählt sind aus Aryl-, insbesondere Phenylresten, und tertiären Alkylresten oder Cycloalkylresten, insbesondere Bicycloalkylresten, wie Adamantyl.

5. Verwendung von Verbindungen der allgemeinen Formel (IV) 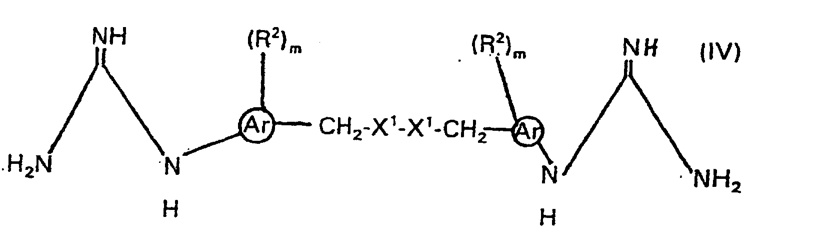 worin
X¹ jeweils unabhängig NR³R⁴, OR³, SR³, COOR³, CONR³R⁴ oder COR⁵ bedeutet, mit der Maßgabe, dass die zwei Arylguanidingruppen über die Substituenten CH₂X¹ miteinander verknüpft sind,
wobei
R³ jeweils unabhängig H oder einen beliebigen organischen Rest bedeutet,
R⁴ jeweils unabhängig H oder einen Alkyl-, Alkenyl- oder Alkinyl-Rest bedeutet,
Ar jeweils unabhängig ein aromatisches oder heteroaromatisches Ringsystem bedeutet,
R² jeweils unabhängig Halogen, C(R⁶)₃ , C₂(R⁶)₅, OC(R⁶)₃ oder OC₂(R⁶)₅ bedeutet,
wobei
R⁶ jeweils unabhängig H oder Halogen, insbesondere F, ist, und
m eine ganze Zahl von 0 bis 4 ist;
oder Salzen dieser Verbindungen zur Herstellung eines Mittels zur Hemmung des Urokinase-Plasminogenaktivators.

6. Verwendung nach einem der Ansprüche 1 bis 5 zur Bekämpfung von Krankheiten, die mit einer pathologischen Überexpression von Urokinase oder/und Urokinase-Rezeptor assoziiert sind.

7. Verwendung nach Anspruch 6 zur Tumorbekämpfung.

8. Verwendung nach Anspruch 6 oder 7 zur Bekämpfung der Metastasenbildung.

9. Verwendung nach einem der vorhergehenden Ansprüche zur Herstellung von oral, topisch, rektal oder parenteral verabreichbaren Arzneimitteln.

10. Verwendung nach einem der vorhergehenden Ansprüche in Form von Tabletten, Dragees, Kapseln, Pellets, Suppositorien, Lösungen oder transdermalen Systemen, wie Pflastern.

11. Verbindungen der Formel (I) 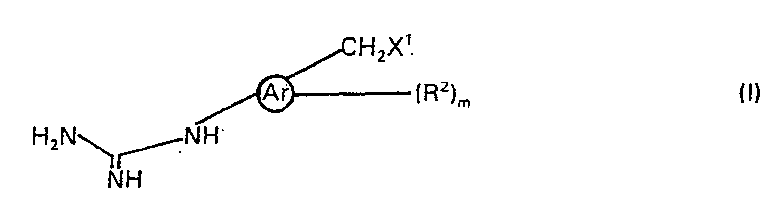 worin Ar, X¹, R² und m wie in einem der Ansprüche 1 bis 5 definiert sind.

## Claims

1. The use of compounds of the formula (I) 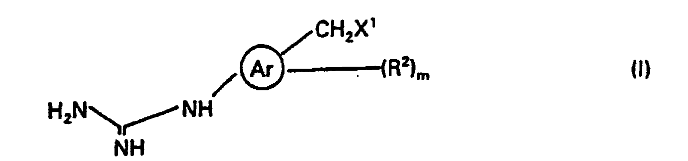 in which
Ar is an aromatic or heteroaromatic ring system having a single ring;
X¹ is NR³R⁴, OR³, SR³, COOR³, CONR³R⁴ or COR⁵,
where
R³ is H or a group of the formula II, IIIa, IIIb or IIIc: 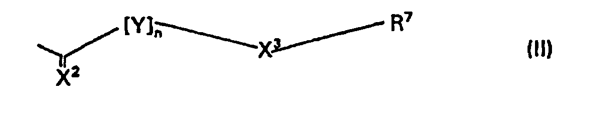 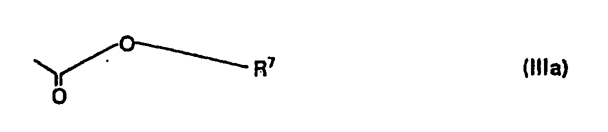 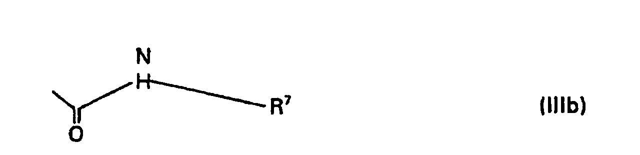 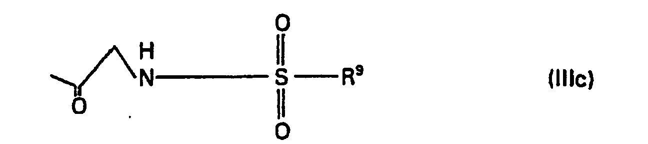 where
X² is NH, NR⁴, O or S,
X³ is NH, NR⁴, O, S, CO, COO, CONH or CONR⁴,
Y is C (R⁸)₂,
R⁴ is H or an alkyl, alkenyl or alkynyl radical,
R⁷ is H or an alkyl, alkenyl, alkynyl, aryl, heteroaryl or/and cycloalkyl radical or -SO₂-R⁹,
R⁸ is in each case independently H, halogen or an alkyl, alkenyl, alkynyl, aryl or/and heteroaryl radical,
R⁹ is H or an alkyl, alkenyl, alkynyl, aryl, heteroaryl or/and cycloalkyl radical and
n is an integer from 0 to 2,
R⁴ is as defined above,
R⁵ is H, an alkyl, alkenyl, alkynyl, carboxyalkyl, carboxyalkenyl, carboxyalkynyl, carboxyaryl or carboxyheteroaryl radical;
R² is halogen, C(R⁶)₃, C₂(R⁶)₅, OC(R⁶)₃ or OC₂(R⁶)₅,
where
R⁶ is in each case independently H or halogen, in particular F; and
m is an integer from 0 to 4;
or salts of said compounds for preparing an agent for inhibition of the urokinase plasminogen activator.

2. The use according to Claim 1, in which Ar is a benzene ring.

3. The use according to Claim 2, in which the substituents -CH₂X¹ and -NHC(NH)NH₂ are arranged in para position.

4. The use according to any of Claims 1 to 3, in which R⁷ and R⁹ are selected from the group comprising aryl, in particular phenyl radicals and tertiary alkyl radicals and cycloalkyl radicals, in particular bicycloalkyl radicals such as adamantyl.

5. The use of compounds of the formula (IV) 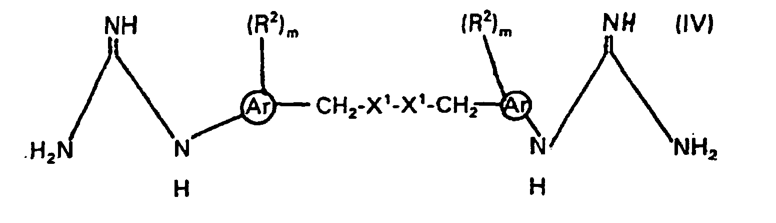 in which
X¹ is in each case independently NR³R⁴, OR³, SR³, COOR³, CONR³R⁴ or COR⁵, with the proviso that the two arylguanidine groups are linked to one another via the substituents CH₂X¹,
where
R³ is in each case independently H or any organic radical,
R⁴ is in each case independently H or an alkyl, alkenyl or alkynyl radical;
Ar is in each case independently an aromatic or heteroaromatic ring system,
R² is in each case independently halogen, C(R⁶)₃, C₂(R⁶)₅, OC(R⁶)₃ or OC₂(R⁶)₅,
where
R⁶ is in each case independently H or halogen, in particular F; and
m is an integer from 0 to 4;
or salts of said compounds for preparing an agent for inhibition of the urokinase plasminogen activator.

6. The use according to any of Claims 1 to 5 for controlling disorders which are associated with a pathological overexpression of urokinase or/and urokinase receptor.

7. The use according to Claim 6 for controlling tumours.

8. The use according to Claim 6 or 7 for controlling the formation of metastases.

9. The use according to any of the preceding claims for preparing orally, topically, rectally or parenterally administrable medicaments.

10. The use according to any of the preceding claims in the form of tablets, coated tablets, capsules, pellets, suppositories, solutions or transdermal systems such as plasters.

11. Compounds of the formula (I) 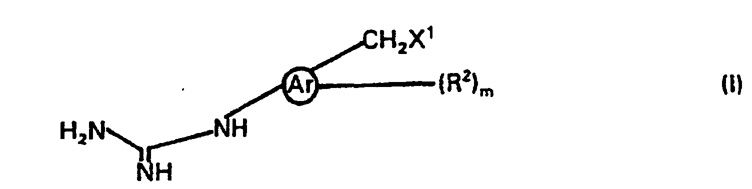 in which Ar, X¹, R² and m are as defined in any of Claims 1 to 5.

## Revendications

1. Utilisation de composés de formule générale (I) 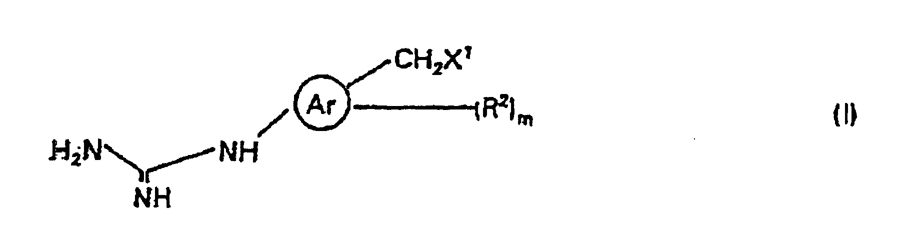 dans laquelle
Ar signifie un système de cyclique aromatique ou hétéroaromatique avec un cycle unique ;
X¹ signifie NR³R⁴, OR³, SR³, COOR³, CONR³R⁴ ou COR⁵,
où
R³ signifie H ou un groupe de formule générale II, IIIa, IIIb ou IIIC : 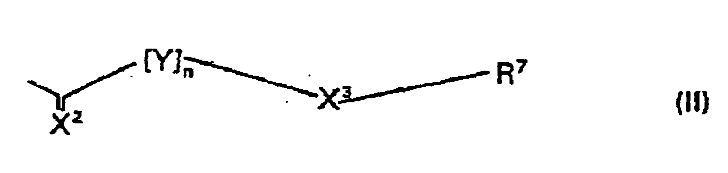 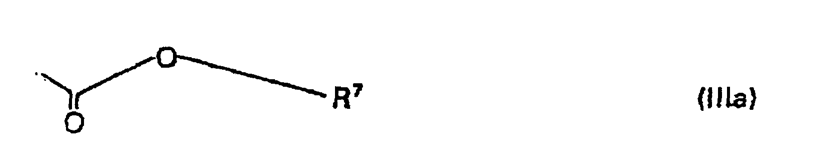 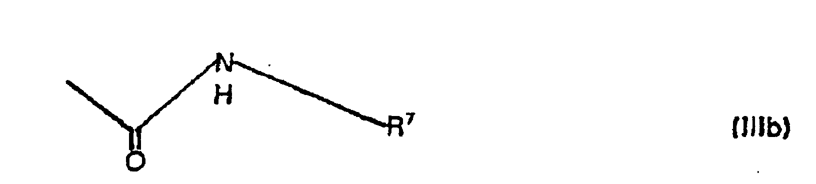 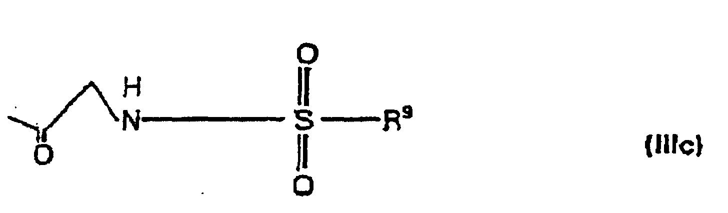 où
X² signifie NH, NR⁴, O ou S,
X³ signifie NH, NR⁴, O, S, CO, COO, CONH ou CONR⁴,
Y signifie C(R⁸)₂,
R⁴ signifie H ou un radical alkyle, alcényle ou alcynyle,
R⁷ signifie H ou un radical alkyle, acényle, alcynyle, aryle, hétéroaryle ou/et cycloalkyle ou signifie -SO₂-R⁹,
R⁸ signifie indépendamment H, un halogène ou un radical alkyle, alcényle, alcynyle, aryle ou/et hétéroaryle,
R⁹ signifie H ou un radical alkyle, alcényle, alcynyle, aryle, hétéroaryle ou/et cycloalkyle et
n est un nombre entier compris allant de 0 à 2,
R⁴ signifie comme défini ci-dessus,
R⁵ signifie H, un radical alkyle, alcényle, alcynyle, carboxyalkyle, carboxy-alcényle, carboxy-alcynyle, carboxy-aryle ou carboxy-hétéroaryle ;
R² signifie un halogène, C(R⁶)₃, C₂(R⁶)₅, OC(R⁶)₃ ou OC₂(R⁶)₅,
où
R⁶ signifie indépendamment un H ou un halogène, en particulier F ;
m est un nombre entier allant de 0 à 4 ;
ou des sels de ces composés pour la fabrication d'un agent pour l'inhibition de l'activateur du plasminogène urokinase.

2. Utilisation selon la revendication 1, dans laquelle Ar est un cycle benzol.

3. Utilisation selon la revendication 2, dans laquelle les substituants -CH₂X¹ et -NHC (NH) NH₂ sont placés en position para l'un par rapport à l'autre.

4. Utilisation selon l'une des revendications 1 à 3, dans laquelle R⁷ et R⁹ sont choisis parmi des radicaux aryle, en particulier phényle, et des radicaux alkyle ou des radicaux cycloalkyle tertiaires, en particulier des radicaux bicycloalkyles, comme l'adamantyle.

5. Utilisation de composés de formule générale (IV) 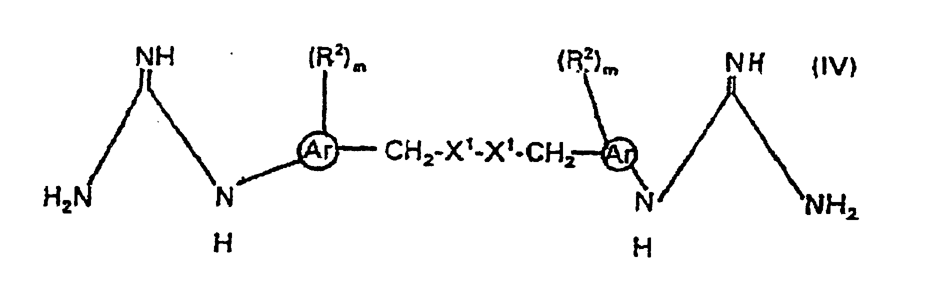 dans laquelle
X¹ signifie indépendamment NR³R⁴ , OR³, SR³, COOR³ , CONR³R⁴ ou COR⁵, à la condition que les deux groupes arylguanidine soient liés par les substituants CH₂X¹,
où
R³ signifie indépendamment H ou un radical organique quelconque,
R⁴ signifie indépendamment H ou un radical alkyle, alcényle ou alcynyle,
Ar signifie indépendamment un système cyclique aromatique ou hétéroaromatique,
R² signifie indépendamment un halogène, C(R⁶)₃, C₂(R⁶)₅, OC(R⁶)₃ ou OC₂(R⁶)₅,
où
R⁶ est indépendamment H ou un halogène, en particulier F et
m est un nombre entier allant de 0 à 4 ;
ou des sels de ces composés pour fabriquer un agent pour l'inhibition de l'activateur du plasminogène urokinase.

6. Utilisation selon l'une des revendications 1 à 5 pour lutter contre des maladies qui sont associées à une surexpression pathologique de l'urokinase ou/et du récepteur de l'urokinase.

7. Utilisation selon la revendication 6 pour lutter contre une tumeur.

8. Utilisation selon la revendication 6 ou la revendication 7 pour lutter contre la formation de métastases.

9. Utilisation selon l'une des revendications précédentes pour la fabrication de médicaments pouvant être administrés par voie orale, topique, rectale ou parentérale.

10. Utilisation selon l'une des revendications précédentes sous la forme de comprimés, de dragées, de capsules, de pellets, de suppositoires, de solutions ou de systèmes transdermiques comme les emplâtres.

11. Composés de formule (I) 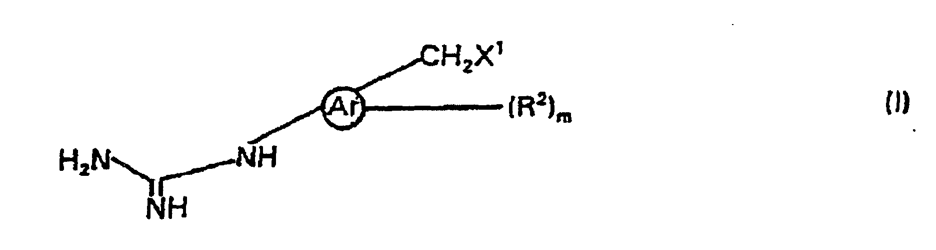 où Ar, X¹, R² et m sont tels que définis dans l'une des revendications 1 à 5.
